# EUROPEAN PATENT APPLICATION

(11) **EP 4 494 653 A1**
(43) Date of publication of application: **22.01.2025**
(21) Application number: 23769537.4
(22) Date of filing: 23.02.2023
(51) Int. Cl.: A61K 39/395, C07K 16/30

(54) **PRLR ANTIGEN-BINDING PROTEIN, PREPARATION METHOD THEREFOR AND USE THEREOF**

(30) Priority: 16.03.2022 CN 202210261642
(71) Applicant: Shenyang Sunshine Pharmaceutical Co., Ltd., Shenyang, Liaoning 110027 (CN)
(72) Inventor: LOU, Jing, Shenyang, Liaoning 110027 (CN); LIU, Ying, Shenyang, Liaoning 110027 (CN); LIU, Zhilan, Shenyang, Liaoning 110027 (CN); CHEN, Baofeng, Shenyang, Liaoning 110027 (CN)
(74) Representative: Chung, Hoi Kan
(86) International application number: PCT/CN2023/077847
(87) International publication number: WO 2023/174029

(57) **Abstract**

Provided are a PRLR antigen-binding protein, a preparation method therefor and use thereof. By means of the hybridoma technology, the PRLR antigen-binding protein with good affinity and binding activity to human PRLR-ECD is obtained, which can effectively inhibit the activation of PRLR. The PRLR antigen-binding protein can be used for treating various PRLR-positive diseases including a tumor and alopecia.

## Description

### TECHNICAL FIELD

The present invention belongs to the field of biomedicine, and specifically relates to a PRLR antigen-binding protein, a preparation method therefor and use thereof.

### BACKGROUND

Prolactin (PRL), also known as lactotropin or lactogenic hormone, is an anterior pituitary peptide hormone necessary for successful reproduction. A prolactin receptor (PRLR) constitutes one gene family with a growth hormone receptor (GHR) and a placental lactogen receptor (PLR). PRLR is a cell membrane receptor expressed in a mammary gland, an ovary, a pituitary gland, a heart, lungs, thymuses, a spleen, a liver, a pancreas, kidneys, adrenal glands, a uterus, a skeletal muscle, skin, and a central nervous system. PRLR is related with a variety of physiological functions, including cell proliferation, differentiation, lactation, and reproduction.

At least two forms of receptors in PRL, that is, a long receptor (a long form) and a short receptor (a short form), are known to exist in a mammal. PRLR structures are diverse and are mainly caused by variation in an intracellular domain. PRLR is a transmembrane protein including only one transmembrane region, which consists of three parts: an extramembrane domain, a transmembrane domain, and an intracellular domain. PRLR has the structural characteristics of a cytokine receptor super family. PRLR consists of two domains, namely, Fibronectin type-III 1 and Fibronectin type-III 2. The difference between PRLR structures of a mammal and an avian is that the mammal has only one extracellular ligand-binding region, whereas the avian has two ligand-binding regions of repetitive units. The genomic structure of a human PRLR gene is well known (Hu, Z.-Z. et al., J. Clin. Endocr. Metab. 84:1153-1156, 1999). A 5-guided-untranslated region of a PRLR gene includes two first exons. E13, the human counterpart of E13 of a rat and a mouse, and a new human-type first exon are termed as E1N. The 5-guided-untranslated region also includes one generic non-coding exon 2 and a portion of an exon 3. The exon 3 includes a translation initiation codon. Exons E13 and ElN are within 800 base pairs. Both of the exons are expressed in a human mammary tissue, a breast cancer cell, a gonad, and a liver. In summary, a transcripton including E13 is expressed in most tissues. A PRLR gene product is encoded by exons 3-10, with an exon 10 encoding the majority of an intracellular region. The exons E13 and ElN start to be transcribed from one of two promoters PIII and PN, respectively. The promoter PIII includes Spl and C/EBP elements that are consistent with those found in a rodent promoter and shares 81% homology with a -480/-106 region of a gene in a rat and a mouse. The promoter PN includes a binding site for a putative ETS family protein and a half-site for a nuclear receptor.

The binding of PRL to a receptor thereof forms a trimer, which leads to the activation of PRL-R, and is a necessary condition and prerequisite for the production of a variety of biological functions. After the activation of PRL-R, JAK2 can be phosphorylated, which induces the phosphorylation of distal tyrosine in a receptor cell and activates a STAT protein. The activated STAT moves into a nucleus to activate the transcription of a target gene and produce a biological effect, which is a basic model for regulating an immune function of PRL as a mediator of a nerve, an endocrine and an immune network. In addition, the binding of PRL to the receptor thereof can activate signal pathways PI3K and MAPK, increase the expression of a cyclin Dl, and promote the growth of a breast cancer cell.

PRLR is highly expressed on a variety of endocrine-related tumor cells, including a breast cancer, a prostate cancer, and an ovarian cancer. PRLR is related with the occurrence and development of these tumors. The elevated circulating concentration of a ligand prolactin (PRL) is a high-risk factor for the occurrence of the breast cancer, and one negatively correlated indicator for determining prognosis for a breast cancer patient. Recent studies have shown that the activation of PRLR is related with alopecia. The elevated circulating concentration of prolactin is strongly related with alopecia. Therefore, PRLR serves as one target for drug development, and effectively inhibit the activation of PRLR, which can treat a variety of related diseases. Patent CN200780030284.7 discloses a PRLR-specific antibody and a pharmaceutical composition including the antibody, a kit including the pharmaceutical composition, and a method for preventing and treating a cancer. Patent CN201480055822.8 also discloses an antibody for binding to the prolactin receptor PRLR and a method for using thereof. The antibody is bound to a human PRLR with high affinity and blocks prolactin-mediated cellular signal transduction, which can be used for the treatment of a variety of cancers as well as other PRLR-related disorders.

However, there are fewer antibodies against PRLR in the prior art. Therefore, there is an urgent need to provide a new high-affinity PRLR-specific antibody that can be bound to PRLR and inhibit the activation of PRLR. Therefore, the antibody can be used for the treatment of a variety of PRLR-related diseases including a tumor and alopecia.

### SUMMARY

In response to the above deficiencies, the present invention provides a new PRLR antigen-binding protein, a preparation method therefor and use thereof. The present invention employs hybridoma technology to obtain a series of new PRLR antigen-binding proteins with biological functions. The PRLR antigen-binding protein has better affinity and binding activity for human PRLR-ECD and inhibits the activation of PRLR. The PRLR antigen-binding protein described in the present invention can be used for the treatment of a variety of PRLR-related diseases, including a tumor and alopecia, and provides a new idea for the treatment of the PRLR-related diseases.

In order to realize the above objects of the invention, the technical solution of the present invention is as follows:

In an aspect, the present invention provides a PRLR antigen-binding protein.

Specifically, the PRLR antigen-binding protein includes the following complementarity-determining regions:
(1) a heavy-chain complementarity-determining region 1HCDR1 including an amino acid sequence shown in SEQ ID NO: 12 or a variant sequence thereof;
(2) a heavy-chain complementarity-determining region 2HCDR2 including an amino acid sequence shown in SEQ ID NO: 13 or a variant sequence thereof;
(3) a heavy-chain complementarity-determining region 3HCDR3 including an amino acid sequence shown in SEQ ID NO:14 or a variant sequence thereof;
(4) a light-chain complementarity-determining region 1LCDR1 including an amino acid sequence shown in SEQ ID NO:15 or a variant sequence thereof;
(5) a light-chain complementarity-determining region 2LCDR2 including an amino acid sequence shown in SEQ ID NO:16 or a variant sequence thereof;
(6) a light-chain complementarity-determining region 3LCDR3 including an amino acid sequence shown in SEQ ID NO:17 or a variant sequence thereof;

Preferably, the variant sequence is a CDR sequence having the substitution, deletion or additions of one or several amino acids as compared to a CDR from which the variant sequence is derived. The substitution is conservative substitution.

Further specifically, the PRLR antigen-binding protein includes:
(1). a heavy-chain variable region VH including an amino acid sequence shown in SEQ ID NO:2; and/or a light-chain variable region VL including an amino acid sequence shown in SEQ ID NO:4;
or (2). a VH having at least 70%, at least 80%, at least 85%, at least 90%, at least 91%, at least 92%, at least 93%, at least 94%, at least 95%, at least 96%, at least 97%, at least 98%, or at least 99% sequence identity as compared to the VH in (1); and/or, a VL having at least 70%, at least 80%, at least 85%, at least 90%, at least 91%, at least 92%, at least 93%, at least 94%, at least 95%, at least 96%, at least 97%, at least 98%, or at least 99% sequence identity compared to the VL in (1);
or (3). a VH having the substitution, deletion or addition of one or several amino acids or any combination thereof as compared to the VH in (1); and/or, a VL having the substitution, deletion or addition of one or several amino acids or any combination thereof as compared to the VL in (1). The substitution is the conservative substitution.

Further specifically, the PRLR antigen-binding protein further includes a human IgGl constant region and a human kappa-chain constant region. The human IgG1 constant region has an amino acid sequence as shown in SEQ ID NO: 6. The human kappa-chain constant region has an amino acid sequence as shown in SEQ ID NO: 7.

Further specifically, the VH of the PRLR antigen-binding protein is linked to the human IgG1 constant region to form a heavy chain. The VL of the PRLR antigen-binding protein is linked to the human kappa-chain constant region to form a light chain.

Further specifically, the PRLR antigen-binding protein includes:
(1) a heavy chain HC including an amino acid sequence shown in SEQ ID NO:8; and/or a light chain LC including an amino acid sequence shown in SEQ ID NO:9;
or (2) a heavy chain and a light chain, where the heavy chain has at least 80%, at least 85%, at least 90%, at least 91%, at least 92%, at least 93%, at least 94%, at least 95%, at least 96%, at least 97%, at least 98%, or at least 99% sequence identity as compared to the heavy chain and the light chain in (1); and/or, the light chain has at least 70%, at least 80%, at least 85%, at least 90%, at least 91%, at least 92%, at least 93%, at least 94%, at least 95% , at least 96%, at least 97%, at least 98% or at least 99% sequence identity.

Further specifically, the PRLR antigen-binding protein includes a chimeric antibody, a humanized antibody, or a fully human antibody.

Further specifically, the above PRLR antigen-binding protein includes a full-length antibody, a Fab, a Fab', a F(ab')2, a Fv, a scFv > di-scFv, a bispecific antibody, a polyspecific antibody, a heavy chain antibody and/or a single-domain antibody, or a monoclonal antibody and/or a polyclonal antibody derived from the above antibodies.

In another aspect, the present invention provides a humanized PRLR antigen-binding protein, including:
(1). a heavy-chain variable region VH including an amino acid sequence shown in SEQ ID NO:18; and/or a light-chain variable region VL including an amino acid sequence shown in SEQ ID NO:19:
or (2). a VH having at least 70%, at least 80%, at least 85%, at least 90%, at least 91%, at least 92%, at least 93%, at least 94%, at least 95%, at least 96%, at least 97%, at least 98%, or at least 99% sequence identity as compared to the VH in (1); and/or, a VL having at least 70%, at least 80%, at least 85%, at least 90%, at least 91%, at least 92%, at least 93%, at least 94%, at least 95%, at least 96%, at least 97%, at least 98%, or at least 99% sequence identity compared to the VL in (1);
or (3). a VH having the substitution, deletion or addition of one or several amino acids or any combination thereof as compared to the VH in (1); and/or, a VL having the substitution, deletion or addition of one or several amino acids or any combination thereof as compared to the VL in (1). The substitution is the conservative substitution

Specifically, the humanized PRLR antigen-binding protein includes:
(1) a heavy chain HC including an amino acid sequence shown in SEQ ID NO:20; and/or a light chain LC including an amino acid sequence shown in SEQ ID NO:21;
or (2) a heavy chain and a light chain, where the heavy chain has at least 80%, at least 85%, at least 90%, at least 91%, at least 92%, at least 93%, at least 94%, at least 95%, at least 96%, at least 97%, at least 98%, or at least 99% sequence identity as compared to the heavy chain and the light chain in (1); and/or, the light chain has at least 70%, at least 80%, at least 85%, at least 90%, at least 91%, at least 92%, at least 93%, at least 94%, at least 95% , at least 96%, at least 97%, at least 98% or at least 99% sequence identity.

In another aspect, the present invention provides a series of nucleic acid molecules for encoding the PRLR antigen-binding protein or a humanized PRLR antigen-binding protein.

Specifically, the nucleic acid molecules include one or more codon-optimized nucleic acid molecules.

In another aspect, the present invention provides a series of vectors. The vectors include one or more nucleic acid molecules as described in the present application.

Specifically, the vectors include, but are not limited to, a plasmid, a virus, and a bacteriophage.

In another aspect, the present invention provides a series of host cells including the above nucleic acid molecules or the above vectors.

Specifically, the host cells include, but are not limited to, microbial, plant or animal cells. The vectors described herein can be introduced into the host cells by a method known to a person skilled in the art, such as electroporation, lipofectine transfection and lipofectamin transfection.

In another aspect, the present invention provides a chimeric antigen receptor, including the above PRLR antigen-binding protein or a humanized PRLR antigen-binding protein.

In another aspect, the present invention provides an immune cell, including the above chimeric antigen receptor.

In another aspect, the present invention provides an antigen-binding protein derivative, including the PRLR antigen-binding protein or a humanized PRLR antigen-binding protein and a detectable labeled molecule.

Specifically, the detectable labeled molecule is an enzyme (e.g. horseradish peroxidase), a radionuclide, a fluorescent dye, a luminescent substance (e.g., a chemiluminescent substance) or a biotin.

In another aspect, the present invention provides a polyspecific antibody, including the above PRLR antigen-binding protein or a humanized PRLR antigen-binding protein, and an additional antibody or a fragment thereof or an antibody mimetic.

Specifically, the polyspecific antibody is a bispecific antibody or a trispecific antibody or a tetraspecific antibody.

In another aspect, the present invention provides an antibody-drug conjugate, including an antibody portion and a coupling portion. The antibody portion includes the above PRLR antigen-binding protein or a humanized PRLR antigen-binding protein. The coupling portion includes, but are not limited to, a detectable label, a drug, a toxin, a cytokine, a radionuclide, an enzyme, or a combination thereof. The antibody portion and the coupling portion are coupled by a chemical bond or a junction.

In another aspect, the present invention provides a pharmaceutical composition, including the above PRLR antigen-binding protein, a humanized PRLR antigen-binding protein, a nucleic acid molecule, a vector, a host cell, a chimeric antigen receptor, an immune cell, an antigen-binding protein derivative, a polyspecific antibody, and/or an antibody-drug conjugate.

Specifically, the pharmaceutical composition also includes an optional pharmaceutically acceptable vector.

Further specifically, the pharmaceutically acceptable vector includes, but is not limited to, a diluent, an excipient, a filler, a wetting agent, a disintegrant, a corrigent, and a binder.

Specifically, the pharmaceutical composition further includes a combination therapeutic agent, including, but not limited to, a chemotherapeutic agent, a radiotherapeutic agent, an immunosuppressive agent, and a cytotoxic drug.

In another aspect, the present invention provides a method for producing the above PRLR antigen-binding protein or a humanized PRLR antigen-binding protein, including the step of: culturing the host cells in a manner that allows expression of the antigen-binding protein.

In another aspect, the present invention provides use of the PRLR antigen-binding protein, a humanized PRLR antigen-binding protein, a nucleic acid molecule, the above vector, a host cell, a chimeric antigen receptor, an immune cell, an antigen-binding protein derivative, a polyspecific antibody, an antibody-drug coupling and/or a pharmaceutical composition in the preparation of a drug for blocking PRLR, a kit and/or a medical device.

Specifically, the PRLR blocking drug, the kit and/or the medical device is primarily applied in a disease with increased PRLR expression quantity.

In another aspect, the present invention provides use of the PRLR antigen-binding protein, a humanized PRLR antigen-binding protein, a nucleic acid molecule, the above vector, a host cell, a chimeric antigen receptor, an immune cell, an antigen-binding protein derivative, a polyspecific antibody, an antibody-drug coupling and/or a pharmaceutical composition in the preparation of a drug for preventing and/or treating a PRLR-positive disease, a kit and/or an administering device.

Specifically, the PRLR-positive disease is a tumor, alopecia or the like.

Further specifically, the tumor includes, but is not limited to: a breast cancer, a prostate cancer, an ovarian cancer, a lung cancer, a skin cancer, a pancreatic cancer, a kidney cancer, a stomach cancer, and the like.

In another aspect, the present invention provides use of the PRLR antigen-binding protein, a humanized PRLR antigen-binding protein and/or an antigen-binding protein derivative in the preparation of a PRLR detection reagent or a kit.

In another aspect, the present invention provides a method for detecting PRLR, including the step of: qualitatively or quantitatively analyzing and detecting PRLR with the above PRLR antigen-binding protein or a humanized PRLR antigen-binding protein. The method is used for non-disease diagnostic or therapeutic purpose.

In another aspect, the present invention provides a method for treating a PRLR-positive related disease. The method is used for administering to a subject in need thereof an effective amount of the above PRLR antigen-binding protein, a humanized PRLR antigen-binding protein, an immune cell, an antigen-binding protein derivative, a polyspecific antibody, an antibody-drug coupling and/or a pharmaceutical composition.

Specifically, the PRLR-positive disease is a tumor, alopecia or the like.

Further specifically, the tumor includes, but is not limited to: a breast cancer, a prostate cancer, an ovarian cancer, a lung cancer, a skin cancer, a pancreatic cancer, a kidney cancer, a stomach cancer, and the like.

In another aspect, the present invention provides a kit, including the PRLR antigen-binding protein, a humanized PRLR antigen-binding protein, a nucleic acid molecule, a vector, a host cell, a chimeric antigen receptor, an immune cell, an antigen-binding protein derivative, a polyspecific antibody, an antibody-drug conjugate and/or a pharmaceutical composition, and, optionally, a specification.

In another aspect, the present invention provides an administrating device, including (1) an infusion module for administering the pharmaceutical composition to a subject in need thereof, and (2) optionally, a drug efficacy monitoring module.

Compared to the prior art, the positive and beneficial effects of the present invention are as follows:
(1) The present invention provides a new PRLR antigen-binding protein. By means of the hybridoma technology, the PRLR antigen-binding protein with good affinity and binding activity to human PRLR-ECD is obtained, which can effectively inhibit the activation of PRLR.
(2) The PRLR antigen-binding protein described in the present invention can be used for treating various PRLR-positive diseases including a tumor and alopecia, providing a new idea for treating the PRLR-positive diseases.

### BRIEF DESCRIPTION OF THE DRAWINGS

FIG. 1 is a graph of the detection results of affinity of a murine-derived monoclonal antibody SPGA02-260 bound to human PRLR-ECD.
FIG. 2 is a graph of the detection results of affinity of a murine-derived monoclonal antibody SPGA02-260 bound to a T47D cell with high expression of human PRLR.
FIG. 3 is a graph of the detection results of the inhibition of PRL signal transduction (STATS phosphorylation) by a murine-derived monoclonal antibody SPGA02-260.
FIG. 4 is a graph of the detection results of the inhibition of PRL signal transduction (ERK1/2 phosphorylation) by a murine-derived monoclonal antibody SPGA02-260.
FIG. 5 is a graph of the detection results of an endocytosis effect caused after murine-derived monoclonal antibody SPGA02-260 is bound to a T47D cell.
FIG. 6 is a graph of the detection results of affinity of a chimeric antibody SPGA02-ch260 bound to human PRLR-ECD.
FIG. 7 is a graph of the detection results of affinity of a chimeric antibody SPGA02-ch260 bound to a T47D cell with high expression of human PRLR.
FIG. 8 is a graph of the detection results of affinity of a chimeric antibody SPGA02-ch260 bound to murine-derived PRLR-ECD.
FIG. 9 is a graph of the detection results of the ability of SPGA02-260 bound to different binding regions of target antigen PRLR-ECD.
FIG. 10 is a graph of the detection results of SPGA02-260 bound to a target antigen PRLR-ECD peptide.
FIG. 11 is a graph of the detection results of a humanized monoclonal antibody SPGA02-hu260 bound to murine-derived PRLR.
FIG. 12 is a graph of the detection results of the inhibition of PRL-induced signal transduction (STATS phosphorylation) by a humanized monoclonal antibody SPGA02-hu260.
FIG. 13 is a graph of the detection results of the inhibition of PRL-induced signal transduction (ERK1/2 phosphorylation) by a humanized monoclonal antibody SPGA02-hu260.
FIG. 14 is a graph of the detection results of the inhibition of GH1-induced signal transduction (STATS phosphorylation) by a humanized monoclonal antibody SPGA02-hu260.
FIG. 15 is a graph of the detection results of the inhibition of GH1-induced signal transduction (ERK1/2 phosphorylation) by a humanized monoclonal antibody SPGA02-hu260.
FIG. 16 is a graph of the detection results of the inhibition of the growth of a BAF3-huPRLR cell by a humanized monoclonal antibody SPGA02-hu260.

### DETAILED DESCRIPTION OF THE EMBODIMENTS

The present invention is described in further detail below in connection with specific embodiments. The following embodiments are not intended to limit the present invention, but only to illustrate the present invention. Experimental methods used in the following embodiments where specific conditions are not indicated in the embodiments are usually in accordance with conventional conditions if not otherwise specified. Materials, reagents and the like used in the following embodiments are commercially available if not otherwise specified.

### Definition of Terms

In order to more readily understand the present invention, certain technical and scientific terms are specifically defined below. Unless otherwise expressly defined herein, all other technical and scientific terms used herein have the meanings commonly understood by a person skilled in the art to which this disclosure belongs.

The three-letter and one-letter codes for amino acids used in the present invention are as described in J. biol. chem, 243, p3558 (1968, 1 UPAC-IUB Committee).

The term "antigen-binding protein" as used in the present invention generally refers to a protein including an antigen-binding portion, and optionally a scaffold or backbone portion that allows the antigen-binding portion to adopt a conformation facilitating the binding of the antigen-binding protein to an antigen. The antigen-binding protein may typically include an antibody light-chain variable region (VL), an antibody heavy-chain variable region (VH), or both of the foregoing. The regions VH and VL may be further distinguished as hypervariable regions known as complementarity-determining regions (CDRs), which are dispersed in a more conserved region known as a framework region (FR or FWR). The heavy-chain variable region and the light-chain variable region include binding structural domains that interact with the antigen. Examples of the antigen-binding protein include, but are not limited to, an antibody, an antigen-binding fragment (Fab, Fab', Fv fragments, F(ab')2, scFv, di-scFv, and/or dAb), an immunoconjugate, a polyspecific antibody (e.g., a bispecific antibody), an antibody fragment, an antibody derivative, an antibody mimetic, a chimeric antigen receptor, a fusion protein or the like, as long as the forgoing exhibit desired antigen-binding activity.

The "antibody" of the present invention refers to an immunoglobulin, which is a tetra peptide chain structure formed by two identical heavy chains and two identical light chains that are linked by an inter chain disulfide bond. The composition and arrangement order of amino acids of constant regions of the heavy chains of the immunoglobulin are different, and therefore the antigenicity of the heavy chains is also different. Accordingly, the immunoglobulin may be categorized into five classes, or isotypes of the immunoglobulin, i.e., IgM, IgD, IgG, IgA, and IgE, whose corresponding heavy chains are µ-chain, δ-chain, γ-chain, α-chain, and £-chain, respectively. The same class of Ig can be further divided into different subclasses according to the compositions of amino acids of a hinge region of Ig and the differences in the number and position of the disulfide bonds of the heavy chain, e.g., IgG can be divided into IgG1, IgG2, IgG3, and IgG4. The light chain is divided into a κ chain or a λ chain based on the difference in a constant region. Each of the five classes of Ig can have the κ chain or the λ chain.

The heavy and light chains of the antibody of which about 110 amino acids near N-terminal have highly variable sequences are the variable regions (Fv regions). The heavy and light chains of the antibody of which the remaining amino acids near C-terminal have relatively stable sequences are the constant regions. The variable region includes three hypervariable regions (HVRs) and four framework regions (FRs) with relatively conservative sequences. The three hypervariable regions determine the specificity of the antibody and are also known as complementarity-determining regions (CDRs). Each light-chain variable region (VL or LCVR) and each heavy-chain variable region (VH or HCVR) both consist of 3 CDRs and 4 FRs in the following order from an amino-terminal to a carboxy-terminal: FR1, CDR1, FR2, CDR2, FR3, CDR3, and FR4. The three CDRs of the light chain refer to LCDR1, LCDR2 and LCDR3. The three CDRs of the heavy chain refer to HCDR1, HCDR2 and HCDR3.

The term "complementarity-determining region" (CDR) refers to one of six hypervariable regions within a variable structural domain of the antibody that primarily contribute to antigen binding. Typically, there are three CDRs (HCDR1, HCDR2, HCDR3) per heavy chain variable region and three CDRs (LCDR1, LCDR2, LCDR3) per light chain variable region. The CDRs may be determined according to various numbering systems known in the art, such as Kabat and Chothia numbering systems, or AbM or IMGT numbering system. The amino acid sequence boundaries of the CDRs are determined in the present invention using the "Kabat numbering rule" (see Kabat et al. (1991)).

The term "framework region" residues or "FR" residues refer to those amino acid residues in the variable region of the antibody other than CDR residues as defined above.

The term "monoclonal antibody" or "mAb" refers to an antibody obtained from a group of substantially homogeneous antibodies, i.e., an epitope constituting the group with the same individual antibody and/or the same binding, except possible variant antibodies. Different from a polyclonal antibody preparation which typically includes different antibodies directed against different determinants, each monoclonal antibody of a monoclonal antibody preparation is directed against a single determinant on an antigen. Therefore, "Mono clone" refers to the characteristic of the antibody obtained from a substantially homogeneous antibody group and is not construed as requiring any particular method to produce the antibody. The monoclonal antibody described herein may be prepared by a variety of techniques known to a person skilled in the art. The techniques include, but are not limited to, a hybridoma method, a recombinant DNA method, a bacteriophage display method, a transgenic method and the like.

The term "humanized monoclonal antibody" refers to an antibody produced by transplanting a murine CDR sequence into a human antibody variable region framework, i.e., a different type of human germline antibody framework sequence, which can overcome a heterologous reaction induced by a chimeric antibody carrying a large number of murine protein components. To avoid the decrease in activity induced along with the decrease in immunogenicity, the minimum reverse mutations (or revertant mutations) can be performed on a human antibody variable region framework sequence to maintain the activity. To prepare the humanized antibody, a murine CDR can be inserted into a human framework sequence using a method known in the art (see U.S. Patent No. 5,225,539 by Winter; U.S. Patents Nos. 5,530,101; 5,585,089; 5,693,762 and 6,180,370 by Queen et al; and Lo, Benny, K.C., editor, in Antibody Engineering. Methods and Protocols, volume 248, Humana Press, New Jersey, 2004). Alternatively, a transgenic animal can be utilized, is capable of not producing an endogenous immunoglobulin following immunization and is capable of producing an intact human antibody library (see, e.g., Jakobovits et al, 1993, Proc. Natl. Acad. Sci. USA 90: 2551; Jakobovits et al, 1993, Nature 362: 255- 258; Bruggermann et al, 1993, Year in Immunology 7:33; and Duchosal et al, 1992, Nature 355:258; Lonberg et al (1994) Nature 368(6474):856-859; WO02/43478). Other methods of antibody humanization and modification include a bacteriophage display technique (Hoogenboom et al. 1991, J. Mol. Biol. 227:381; Marks et al. 1991, J. Mol. Biol. 222:581-597; Vaughan et al. 1996, Nature Biotech 14:309).

The terms "specific binding" and "selective binding" refer to a non-random binding reaction between two molecules, such as a reaction between the antibody and the antigen against which the antibody is directed. The strength or affinity of a specific binding interaction can be expressed with a dissociation equilibrium constant (KD) of interaction. As used herein, the term "KD" refers to the dissociation equilibrium constant for a particular antibody-antigen interaction, which is used to describe binding affinity between the antibody and the antigen. The smaller the dissociation equilibrium constant, the tighter the antibody-antigen binding and the higher the affinity between the antibody and the antigen. Typically, the antibodies are bound with an affinity (KD) of about less than 10⁻⁸ M, such as about less than 10⁻⁹ M, 10⁻¹⁰ M, 10⁻¹¹ M or less.

The term "identity" generally refers to the percentage of amino acid residues or a nucleotide in a query sequence to an identical residue in a second reference polypeptide sequence or a portion thereof after comparison of the sequences. A vacancy (GAPS) is introduced where necessary to achieve the maximum percentage of sequence identity, without regard to any conserved substitution as a part of the sequence identity. The comparison used to determine the percentage of amino acid/nucleic acid sequence identity can be accomplished in various ways known in the art, e.g., using publicly available computer software such as BLAST, BLAST-2, ALIGN, NEEDLE or Megalign (DNASTAR). A person skilled in the art can determine suitable parameters for measuring the comparison, including any algorithm required to achieve the maximum comparison over the full length of the sequences compared. An identity percentage may be determined over the entire length of an identified polypeptide/polynucleic acid sequence, or may be determined over a shorter length, such as the length of a fragment obtained from a larger identified polypeptide/polynucleic acid sequence. It is understood that the length of any fragment supported by a sequence shown herein in a table, a figure, or a sequence list may be used as the length of a measurable identity percentage. A sequence having "% identity" retains an important biological activity, such as antibody binding specificity, of a sequence with which the sequence having "% identity" is compared or from which the sequence having "% identity" is derived. A sequence having the substitution, deletion or addition of one or several amino acids, or any combination thereof retains significant biological activity, such as the antibody binding specificity, of a sequence with which the sequence having the substitution, deletion or addition of one or several amino acids, or any the combination thereof is compared or from which the sequence having the substitution, deletion or addition of one or several amino acids, or any combination thereof is derived. A nucleic acid sequence having a "% identity" or a nucleic acid sequence differing by no more than 3, 6, 15, 30 or 45 nucleic acids is capable of performing a function similar to that of a nucleic acid sequence with which the nucleic acid sequence having the "% identity" or the nucleic acid sequence differing by no more than 3, 6, 15, 30 or 45 nucleic acids is compared or from which the nucleic acid sequence having the "% identity" or the nucleic acid sequence differing by no more than 3, 6, 15, 30 or 45 nucleic acids is derived, e.g., expressed proteins are all capable of binding specifically to the same antigen or molecule.

The term "conservative substitution" refers to an amino acid substitution that does not adversely affect or alter the intended property of the protein/polypeptide including the amino acid sequence. The conservative substitution can be introduced, for example, by a standard technique known in the art such as site-directed mutagenesis and PCR-mediated mutagenesis. The conservative substitution of the amino acid includes substitution in which an amino acid residue is substituted with an amino acid residue having a similar side chain, such as substitution with a residue that is physically or functionally similar to the corresponding amino acid residue (e.g., having a similar size, shape, charge, and chemical property including the ability to form a covalent or a hydrogen bond). A family of the amino acid residue having the similar side chain is defined in the art. These families include an amino acid residue having a basic side chain (e.g., lysine, arginine, and histidine), an acidic side chain (e.g., aspartic acid and glutamic acid), an uncharged polar side chain (e.g., glycine, asparagine, glutamine, serine, threonine, tyrosine, cysteine, and tryptophan), a non-polar side chain (e.g., alanine, valine, leucine, isoleucine, proline, phenylalanine, and methionine), a beta-branched side chain (e.g., threonine, valine, isoleucine) and an aromatic side chain (e.g., tyrosine, phenylalanine, tryptophan, histidine). Thus, it is preferred to substitute the corresponding amino acid residue with another amino acid residue from the same side chain family. A method for identifying conserved substitution of an amino acid is well known in the art (see, e.g., Brummell et al. Biochem.32:1180-1187 (1993); Kobayashi et al. Protein Eng. 12(10):879-884 (1999); and Burks et al. Proc. Natl Acad. Set USA 94:412-417 (1997), which are incorporated herein by reference).

The term "vector" refers to a nucleic acid transport tool into which a polyribonucleic acid can be inserted. The vector is called an expression vector when the vector enables the protein encoded by the inserted polynucleic acid to obtain expression. The vector can be introduced into a host cell by transformation, transduction or transfection so that a genetic element that the vector carries in the host cell obtains expression. The vector is well known to a person skilled in the art and includes, but is not limited to: a plasmid; a phage; a cosmid; an artificial chromosome, such as a yeast artificial chromosome (YAC), a bacterial artificial chromosome (BAC), or a Pl-derived artificial chromosome (PAC); a bacteriophage such as a λ bacteriophage or an M13 bacteriophage and an animal virus. The animal virus that may be used as the vector includes, but is not limited to, a retrovirus (including a lentivirus), an adenovirus, an adeno-related virus, a herpesvirus (e.g., a herpes simplex virus), a poxvirus, a baculovirus, a papillomavirus, and a papillomultiple vacuolated virus (e.g., SV40). A vector may include a variety of elements that control expression. The elements include, but are not limited to, a promoter sequence, a transcription initiation sequence, an enhancer sequence, a selection element, and a reporter gene. Additionally, the vector may include a replication initiation site.

The term "host cell" generally refers to an individual cell, a cell line, or a cell culture that can include or has included the plasmid or the vector including a nucleic acid molecule as described herein, or that is capable of expressing the antibody or an antigen-binding fragment thereof as described herein. The cell may include a progeny of a single host cell. Due to a natural, accidental or intentional mutation, a progeny cell may not necessarily be morphologically or genomically identical to an original parental cell, but is able to express the antibody or the antigen-binding fragment thereof as described in the present application. The cell may be obtained by in vitro transfection of a cell using the vector described in the present application. The cell may be a prokaryotic cell (e.g., E. coli) or a eukaryotic cell (e.g., a yeast cell, such as, a COS cell, a Chinese hamster ovary (CHO) cell, a HeLa cell, a HEK293 cell, a COS-I cell, a NS0 cell or a myeloma cell). In some situations, the cell may be a mammalian cell. For example, the mammalian cell may be a CHOK1 cell.

The term "pharmaceutically acceptable vector" refers to the vector that is pharmacologically and/or physiologically compatible with a subject and an active ingredient, is well known in the art (see, e.g., Remington's Pharmaceutical Sciences. Edited by Gennaro AR, 19th ed. Pennsylvania: Mack Publishing Company, 1995) and includes, but is not limited to: a pH regulator, a surfactant, an adjuvant, an ionic strength enhancer, a diluent, an osmolality-maintaining reagent, a delayed absorption reagent, and a preservative. For example, the pH regulator includes, but is not limited to, a phosphate buffer. The surfactant includes, but is not limited to, a cationic surfactant, an anionic surfactant, or a nonionic surfactant, such as Tween-80. The ionic strength enhancer includes, but is not limited to, sodium chloride. The preservative includes, but is not limited to, various anti-bacterial reagents and anti-fungal reagents, such as parabens, chloretone, phenol and sorbic acid. The osmolality-maintaining reagent includes, but is not limited to, sugar, NaCl and a mimetic thereof. The delayed absorption reagent includes, but is not limited to, monostearate and gelatin. The diluent includes, but is not limited to, water, an aqueous buffer (e.g., a buffered saline), an alcohol, a polyol (e.g., glycerol) and the like. The preservative includes, but is not limited to, various anti-bacterial reagents and anti-fungal reagents, such as thiomersalate, 2-phenoxyethanol, parabens, chloretone, phenol and sorbic acid. A stabilizer has the meaning commonly understood by a person skilled in the art, is capable of stabilizing the desired activity of an active ingredient in a drug and includes, but is not limited to, monosodium glutamate, gelatin, SPGA, sugars (e.g., sorbitol, mannitol, starch, sucrose, lactose, dextran, or glucose), the amino acid (e.g., glutamate and glycine), a protein (e.g., dried whey, albumin, or casein), or a degradation product thereof (e.g., lactalbumin hydrolysate), and the like.

The term "pharmaceutical composition" generally refers to a formulation in a form which permits the biological activity of an active ingredient to be effective and does not include an additional ingredient which is unacceptably toxic to a subject to whom the composition is to be administered. The composition is sterile.

The term "subject" refers to a mammal, e.g., a primate mammal, e.g., a non-human primate mammal, or a human. In some embodiments, the subject (e.g., a human) has a disease such as a tumor or alopecia (the tumor includes, but is not limited to: a breast cancer, a prostate cancer, an ovarian cancer, a lung cancer, a skin cancer, a pancreatic cancer, a kidney cancer, a stomach cancer, and the like), or is at risk of having the disease.

The term "effective amount" means an amount sufficient to obtain, or at least partially obtain, a desired effect. For example, an effective amount for the prevention of a disease (e.g., PRLR-positive related) means an amount sufficient to prevent, stop, or delay the onset of the disease (e.g., a PRLR-positive related disease). An effective amount for the treatment of the disease means an amount sufficient to cure, or at least partially stop, the disease and a complication thereof in a patient who already has the disease. The determination of such an effective amount is well within the competence of a person skilled in the art. For example, the amount effective for therapeutic use depends on the severity of the disease to be treated, the general state of a patient's own immune system, the general condition of the patient such as age, weight, and gender, the manner in which a drug is administered, and other treatments administered concurrently, among other things.

The term "chimeric antigen receptor" means that a chimeric antigen receptor T cell is coupled in vitro to one chimeric protein by an antigen-binding site of an antibody that recognizes an antigen and the intracellular portion of a CD3-δ chain or FcεRIγ, and transfects the patient's T cell by gene transduction to express the chimeric antigen receptor, such that the patient's T cell is "recoded" to be able to generate a large number of specific CAR-T cells. When the recoded chimeric antigen receptor T cell is added to the patient's body, the chimeric antigen receptor acts like a GPS to specifically track, recognize and guide the T cell to kill a cell. Most chimeric antigen receptors consist of an extracellular antigen-binding region (consisting of the light and heavy chains derived from the monoclonal antibody, and joined in a middle by a resilient hinge region to form a single-chain antibody), a transmembrane region, and an intracellular signal transduction region. A CAR structure is obtained in vitro by genetic recombination of scFv, which recognizes an antigen of interest, and an intracellular signal domain "immunoreceptor tyrosine activation motif'.

The term "immune cell" includes a cell of hematopoietic origin that play a role in an immune response, such as a lymphocyte, e.g., a B-cell and the T-cell; a natural killer cell; and a myeloid cell, e.g., a monocyte, a macrophage, an eosinocyte, a mastocyte, a basophil, and a granulocyte.

### Abbreviations

CDR: complementarity-determining region in immunoglobulin variable region.
VH: antibody heavy-chain variable region.
VL: antibody light-chain variable region.
HC: antibody heavy chain.
LC: Antibody light chain.
IgG: Immunoglobulin G.
AbM: The AbM CDR definition is derived from Martin's related study (Martin ACR, Cheetham JC, Rees AR (1989) Modelling antibody hypervariable loops. A combined algorithm. Proc Natl Acad Sci USA 86:9268-9272), which integrates part of the definitions of both Kabat and Chothia.
Kabat: Immunoglobulin comparison and numbering system proposed by Elvin A. Kabat (see, e.g., Kabat et al., Sequences of Proteins of Immunological Interest, 5th Ed. Public Health Service, National Institutes of Health, Bethesda, MD. Institutes of Health, Bethesda, Md., 1991).
Chothia: an immunoglobulin numbering system proposed by Chothia et al. which is a classical rule for identifying a CDR region boundary based on the position of a structural loop region (see, e.g., Chothia & Lesk (1987) J. Mol. Biol. 196:901-917; Chothia et al. (1989) Nature 342:878 -883).
IMGT: a numbering system based on the international ImMunoGeneTics information system^{®} (IMGT) initiated by Lefranc et al. See Lefrancetal., Dev. Comparat. Immunol. 27:55-77, 2003.
mAb: monoclonal antibody.
EC50: Concentration that produces 50% efficacy or binding.
IC50: Concentration that produces 50% inhibition.
ELISA: enzyme-linked immunosorbent assay.
PCR: polymerase chain reaction.
HRP: horseradish peroxidase.
PRLR: prolactin receptor.
hFc: human IgG antibody FC segment.
KD: dissociation equilibrium constant.
HCDR1: a complementarity-determining region 1 in a variable region of immunoglobulin heavy chain.
HCDR2: a complementarity-determining region 2 in a variable region of immunoglobulin heavy chain.
HCDR3: a complementarity-determining region 3 in a variable region of immunoglobulin heavy chain.
LCDR1: a complementarity-determining region 1 in a variable region of an immunoglobulin light chain .
LCDR2: a complementarity-determining region 2 in a variable region of an immunoglobulin light chain .
LCDR3: a complementarity-determining region 3 in a variable region of an immunoglobulin light chain .

### Detailed description of the embodiments

### An antigen-binding protein

The present invention prepares a series of PRLR antigen-binding proteins which are prepared by an immunization mouse, molecular biology and an antibody engineering technique.

In some embodiments, the antigen-binding protein provided by the present invention includes the following complementarity-determining regions:
(1) a heavy-chain complementarity-determining region 1HCDR1 including an amino acid sequence shown in SEQ ID NO: 12 or a variant sequence thereof;
(2) a heavy-chain complementarity-determining region 2HCDR2 including an amino acid sequence shown in SEQ ID NO: 13 or a variant sequence thereof;
(3) a heavy-chain complementarity-determining region 3HCDR3 including an amino acid sequence shown in SEQ ID NO: 14 or a variant sequence thereof;
(4) a light-chain complementarity-determining region 1LCDR1 including an amino acid sequence shown in SEQ ID NO: 15 or a variant sequence thereof;
(5) a light-chain complementarity-determining region 2LCDR2 including an amino acid sequence shown in SEQ ID NO: 16 or a variant sequence thereof;
(6) a light-chain complementarity-determining region 3LCDR3 including an amino acid sequence shown in SEQ ID NO: 17 or a variant sequence thereof;

Preferably, the variant sequence is a CDR sequence having substitution, deletion or additions of one or several amino acids as compared to a CDR from which the variant sequence is derived. The substitution is conservative substitution.

In some embodiments, the antigen-binding protein provided by the present invention includes a heavy-chain variable region VH and a light-chain variable region VL as follows:
(1). a heavy-chain variable region VH including an amino acid sequence shown in SEQ ID NO:2; and/or a light-chain variable region VL including an amino acid sequence shown in SEQIDN0:4;
or (2). a VH having at least 70%, at least 80%, at least 85%, at least 90%, at least 91%, at least 92%, at least 93%, at least 94%, at least 95%, at least 96%, at least 97%, at least 98%, or at least 99% sequence identity as compared to the VH in (1); and/or, a VL having at least 70%, at least 80%, at least 85%, at least 90%, at least 91%, at least 92%, at least 93%, at least 94%, at least 95%, at least 96%, at least 97%, at least 98%, or at least 99% sequence identity compared to the VL in (1);
or (3). a VH having the substitution, deletion or addition of one or several amino acids or any combination thereof as compared to the VH in (1); and/or, a VL having the substitution, deletion or addition of one or several amino acids or any combination thereof as compared to the VL in (1). The substitution is the conservative substitution

In some embodiments, the antigen-binding proteins provided by the present invention includes a heavy chain HC and a light chain LC as follows:
(1) a heavy chain HC including an amino acid sequence shown in SEQ ID NO:8; and/or a light chain LC including an amino acid sequence shown in SEQ ID NO:9;
or (2) a heavy chain and a light chain, where the heavy chain has at least 80%, at least 85%, at least 90%, at least 91%, at least 92%, at least 93%, at least 94%, at least 95%, at least 96%, at least 97%, at least 98%, or at least 99% sequence identity as compared to the heavy chain and the light chain in (1); and/or, the light chain has at least 70%, at least 80%, at least 85%, at least 90%, at least 91%, at least 92%, at least 93%, at least 94%, at least 95%, at least 96%, at least 97%, at least 98% or at least 99% sequence identity.

In certain specific embodiments, the PRLR antigen-binding protein obtained by the preparation of the present invention includes a combination of heavy and light chains as described in Table 1 below.

**Table 1. Sequence characteristics of the PRLR antigen-binding protein**

| Sequence No. | SPGA02-260 |
|---|---|
| HCDR1 | SEQ ID NO:12 |
| HCDR2 | SEQ ID NO:13 |
| HCDR3 | SEQ ID NO:14 |
| LCDRl | SEQ ID NO:15 |
| LCDR2 | SEQ ID NO:16 |
| LCDR3 | SEQ ID NO:17 |
| mVH | SEQ ID NO:2 |
| mVL | SEQ ID NO:4 |
| Human IgG1 constant region | SEQ ID NO:6 |
| Human kappa-chain constant region | SEQ ID NO:7 |
| ChHC | SEQ ID NO:8 |
| ChLC | SEQ ID NO: 9 |
| huVH | SEQ ID NO:18 |
| huVL | SEQ ID NO:19 |
| huHC | SEQ ID NO:20 |
| huLC | SEQ ID NO:21 |

### An antibody derivative and a polyspecific antibody

The antigen-binding protein provided by the present invention as described in the preceding section includes an antibody or an antigen-binding fragment, which may be derivatized (e.g., linked to another molecule, such as another peptide or protein). Typically, derivatization (e.g., labeling) of the antibody or the antigen-binding fragment thereof does not adversely affect the binding of the antibody or the antigen-binding fragment thereof to PRLR. Accordingly, the antibody or the antigen-binding fragment thereof of the present invention is also intended to include a form of such derivatization. For example, the antibody or the antigen-binding fragment thereof of the present invention can be linked to one or more other molecular groups to form a bispecific antibody, an assay reagent, a medicinal reagent, and/or a protein or a polypeptide (e.g., an anti-biotin protein or a polyhistidine tag) that is capable of mediating the binding of the antibody or the antigen-binding fragment to another molecule.

One type of a derivatized antibody is a labeled antibody. For example, the antibody or the antigen-binding fragment thereof of the present invention may be linked to a detectable label. The detectable label described herein may be any substance detectable by fluorescence, spectroscopy, photochemistry, biochemistry, immunology, electricity, optics or chemical means. Such label is well known in the art, examples of which include, but are not limited to, an enzyme (e.g., horseradish peroxidase, alkaline phosphatase, β-galactosidase, urease and glucose oxidase), a radionuclide (e.g., 3H, 125I, 35S, 14C, or 32P), a fluorescent dye (e.g., fluorescein isothiocyanate (FITC), a fluorescein, tetramethylrhodamine isothiocyanate (TRITC), phycoerythrin (PE), Texas red, rhodamine, a quantum dot or a cyanine dye derivative (e.g., Cy7, and Alexa 750)), an acridzha ester compound, a magnetic bead (e.g., Dynabeads^{®}), a calorimetric label such as a colloidal gold, a tinted glass or a plastic (e.g., polystyrene, polypropylene, and latex) bead, and a biotin for combining the above labels to modify an avid in (e.g., streptavid in). Patents teaching the use of such labels include, but are not limited to, U.S. Patents 3817837, 3850752, 3939350, 3996345, 4277437, 4275149, and 4366241 (all of which are incorporated herein by reference). Detectable labels as described above may be detected by a method known in the art. For example, a radioactive label may be detected using a photographic film or a scintillation calculator. A fluorescent label may be detected using a light detector to detect emitted light. An enzymatic label is generally detected by providing the enzyme with a substrate and detecting a reaction product produced through the action of the enzyme on the substrate. The calorimetric label is detected by simply visualizing a coloring label. In some embodiments, such labels can be suitable for immunological detection (e.g., an enzyme-linked immunoassay, a radioimmunoassay, a fluorescent immunoassay, and a chemiluminescent immunoassay). In some embodiments, a detectable label as described above can be linked to the antibody or the antigen-binding fragment thereof of the present invention by means of a linker of varying length to reduce potential site-blocking.

In addition, the antibody or the antigen-binding fragments thereof of the present invention may be derivatized with a chemical group, such as polyethylene glycol (PEG), methyl or ethyl, or a sugar group. These groups may be used to improve the biological property of the antibody, for example, to increase serum half-life.

As another antibody derivative, the present invention provides a polyspecific antibody, including a first antibody or a fragment thereof, and an additional antibody or a fragment thereof, or an antibody mimetic. The first antibody or the fragment thereof, the additional antibody or the fragment thereof, or the antibody mimetic retains an original binding specificity thereof. The first antibody or the fragment thereof is any TAA-binding (monoclonal) antibody or an antigen-binding fragment thereof of the present invention. As used herein, the "antibody mimetic" refers to a substance that binds antigen specifically like the antibody, but does not have an antibody structure. The antibody mimetic is typically an artificial peptide or a protein with a molar mass of about 3 to 20 kDa, such as an anchor protein repeat protein (DARPin) and fynomer. A designed anchor protein repeat protein (DARPin) can be linked to an IgG antibody, a scFv-Fc antibody fragment, or a combination thereof, as described in CN104341529A. Fusion of an anti-IL-17a fynomer with an anti-IL-6R antibody produces a bispecific fusion polypeptide, as described in WO2015141862A1.

In some embodiments, the polyspecific antibody is formed by coupling the first antibody or the antigen-binding fragment thereof with other antibodies or antigen-binding fragments thereof or antibody mimetics thereof. Each of the antibodies or the antigen-binding fragments thereof or the antibody mimetics maintain the original binding specificity thereof. The first antibody or the antigen-binding fragment thereof is the antibody or the antigen-binding fragment thereof as described herein. In some embodiments, the polyspecific antibody is the bispecific antibody or a trispecific antibody or a tetraspecific antibody.

### An antibody-drug conjugate

An antibody drug conjugate includes an antigen-binding protein portion and a coupling portion as provided herein. The antigen-binding protein portion includes the "antigen-binding protein" as described above, and the antigen-binding fragment obtained on the basis of the "antigen-binding protein". Examples of the antigen-binding fragment includes, but is not limited to, Fab, Fab, a Fv fragment, F(ab,)2, scFv, di-scFv and/or dAb, among others. The coupling portion can include at least one payload drug. The payload drug can include a pharmaceutically active substance ingredient and/or a labeling molecule as previously described. The payload drug is a class of pharmacologically active small molecule compounds or a toxin or other forms of drug molecules, which can be, but are not limited to, a small molecule compound, a toxin molecule, an oligonucleotide acid, a protein degradation targeting chimera (PROTAC), an affinity ligand, a fluorescent group, a nuclide group, and the like. A variety of payload drugs are known in the art. For example, the small molecule compound typically refers to a class of substances that are highly cytotoxic. An exemplary small molecule compound may exert such cytotoxic and cytostatic effects through mechanisms including, but not limited to, microtubule protein binding, DNA binding, inhibition of RNA polymerase, protein synthesis, or inhibition of a topoisomerase. For example, the small molecule compound can be a microtubule protein inhibitor; for example, the microtubule protein inhibitor can be maytansine (e.g., DM1 or DM4) and Auristatin (e.g., MMAE or MMAF), among others. For example, the small molecule compound can be a DNA damaging agent; for example, the DNA damaging agent can be calicheamicins, Pyrrolobenzodiazepines (PBD) and the like. For example, the protein degradation-targeted chimera (PROTAC) is a class of compounds capable of causing degradation of a target protein by inducing polyubiquitination of the target protein; for example, the PROTAC may be a BET protein degrader. The drug conjugate can further include at least one linker. For example, the linker may include a breakable linker or a non-breakable linker. The linker is used to link one or more payload drugs to the antigen-binding protein. In the present application, the breakable linker can be a "cleavable" linker that facilitates the release of a drug. For example, a cleavable linker can include, but is not limited to, an acid-sensitive linker, a protease-sensitive linker, a light-sensitive linker, or a disulfide-containing linker. The linker may include one or more linker members. A variety of linker members are known in the art, such as, for example, maleimidocaproyl (MC), maleimido propanoyl (MP), valine-citrulline (Val-Cit or vc), and Paminobenzyloxycarbonyl (PAB).

### Preparation of a nucleic acid, a vector, a host cell and an antibody

The antigen-binding proteins of the present invention may be prepared by various methods known in the art, such as by a genetically engineered recombinant technique. For example, a DNA molecule encoding heavy and light chain genes of the antibody of the present invention are obtained by chemical synthesis or PCR amplification. The resulting DNA molecule is inserted into an expression vector and then transfected into the host cell. The transfected host cell is then cultured under specific conditions and expresses the antibody of the present invention.

In certain specific embodiments, the present invention provides an isolated nucleic acid molecule, including a nucleic acid sequence encoding the antibody or the antigen-binding fragment thereof of the present invention, or a heavy chain variable region thereof and/or a light chain variable region thereof, or one or more CDRs thereof. In some embodiments, the nucleotide sequence is substitutable according to codon degeneracy, as known in the art. In some embodiments, the nucleotide sequence is codon-optimized.

In some embodiments, the present invention provides a cloning vector or an expression vector, including the isolated nucleic acid molecule of the present invention. In some embodiments, the vector is, for example, a plasmid, a cosmid, a bacteriophage, and a lentivirus. In some embodiments, the vector is capable of expressing the antibody or the antigen-binding fragment thereof of the present invention in a subject (e.g., a mammal, e.g., a human). In some embodiments, the present invention provides the host cell including the isolated nucleic acid molecule of the present invention or the vector of the present invention. The host cell may be a eukaryotic cell (e.g., a mammalian cell, an insect cell, and a yeast cell) or a prokaryotic cell (e.g., E. coli). The suitable eukaryotic cell includes, but is not limited to, an NS0 cell, a Vero cell, a Hela cell, a COS cell, a CHO cell, a HEK293 cell, a BHK cell, and an MDCKII cell. The suitable insect cell includes, but is not limited to, an Sf9 cell. In some embodiments, the host cell of the present invention is a mammalian cell, such as a CHO (e.g., CHO-K1, CHO-S, and CHO DXB 11 CHO DG44).

In some embodiments, the present invention provides a method for preparing an antibody or an antigen-binding fragment thereof of the present invention, including the steps of, culturing a host cell of the present invention under conditions that allow expression of the antibody or the antigen-binding fragment thereof, and recovering the antibody or the antigen-binding fragment thereof from a cultured host cell culture.

### Pharmaceutical use, a therapeutic method, a pharmaceutical composition and an administrating device

The present invention provides use of the PRLR antigen-binding protein, an antigen-binding protein derivative, a polyspecific antibody, an immune cell, and an antibody-drug conjugate in the preparation of a drug for blocking PRLR, and a drug for preventing and/or treating a PRLR-positive disease.

Accordingly, the present invention provides a method for blocking PRLR and preventing and/or treating PRLR-positive disease with the PRLR antigen-binding protein, an antigen-binding protein derivative, a polyspecific antibody, an immune cell, and an antibody-drug conjugate.

In some embodiments, the present invention provides a pharmaceutical composition for use in therapy, including the PRLR antigen-binding protein, a nucleic acid molecule, a vector, a host cell, an immune cell, an antigen-binding protein derivative, a polyspecific antibody and/or an antibody-drug conjugate, and optionally a pharmaceutically acceptable vector. The "pharmaceutically acceptable vector" includes, but is not limited to: a diluent, an excipient, a filler, a wetting agent, a disintegrant, a corrigent, and a binder.

In some embodiments, the pharmaceutical composition further includes a combination therapeutic agent. The combination therapeutic agent includes, but is not limited to, a chemotherapeutic agent, a radiotherapeutic agent, an immunosuppressive agent, and a cytotoxic drug.

In some embodiments, in the pharmaceutical composition, the antigen-binding protein of the present invention is provided with the combination therapeutic agent as a separate component or as a component of the same composition. Thus, the antigen-binding protein of the present invention and the combination therapeutic agent may be administered in combination or separately, simultaneously or sequentially. An anti-PRLR Antibody or an antigen-binding fragment thereof may be administered alone or in combination with other therapeutic agents. The anti-PRLR antibody or the antigen-binding fragment thereof and one or more other therapeutic agents may be administered separately, simultaneously or sequentially.

The pharmaceutical composition may be in any suitable form (depending on the desired method of administering the pharmaceutical composition to a patient), and the PRLR antibody of the present invention may be administered to the patient by a variety of routes (e.g., orally, transdermally, subcutaneously, intranasally, intravenously, intramuscularly, intraocularly, topically, intrathecally, and intracerebroventricularly). The most suitable route of administration in any given situation depends on a particular antibody, the nature and severity of an individual and a disease, and the physical condition of the individual.

In some embodiments, the present invention also provides an administrating device for administering the antigen-binding protein, an antigen-binding protein derivative, a polyspecific antibody, an immune cell, an antibody-drug conjugate, and a pharmaceutical composition including the foregoing, including:
(i) an infusion module, configured to administer to a subject a pharmaceutical composition having an active ingredient;
(ii) a pharmaceutical composition for infusion, including an active ingredient, the active ingredient being selected from a group consisting of: an antigen-binding protein, an antigen-binding protein derivative, a polyspecific antibody, an immune cell, an antibody-pharmaceutical conjugate, or a combination thereof; and
(iii) optionally a pharmacophore monitoring module.

In another preferable example, the administration includes parenteral administration and non-parenteral administration. In another preferable example, the parenteral administration includes injectable administration. The route of injection used includes: intravenous, intramuscular, intra-arterial, intra-membranous, intra-capsular, intra-orbital, intra-cardiac, intradermal, intra-peritoneal, trans-tracheal, subcutaneous, sub-epidermal, intra-articular, sub-capsular, sub-arachnoid, intraspinal, supradural and intrasternal injections and push injections. In another preferable example, the non-parenteral administration includes topical, epidermal, or mucosal administration, such as intranasal, transoral, vaginal, rectal, sublingual, or topical administration. In another preferable example, the infusion module is a needleless hypodermic injection device, a microinfusion pump, a transdermal administrating device, a push injection device, or an osmotic device.

### A detection method and a kit

The antibody or the antigen-binding fragments thereof of the present invention is capable of binding PRLR, and thus can be used to detect the presence of PRLR or a level thereof in a sample.

In one aspect, the present invention provides a kit, including an antigen-binding protein of the present invention. In some embodiments, the antigen-binding protein of the present invention bears a detectable label. In one preferable embodiment, the kit further includes a second antibody, which specifically identifies the antibody or the antigen-binding fragment thereof of the present invention. Preferably, the second antibody further includes the detectable label.

In the present invention, the detectable label can be any substance detectable by fluorescent, spectroscopic, photochemical, biochemical, immunological, electrical, optical or chemical means. Particularly preferably, such label is capable of being applied to an immunological assay (e.g., an enzyme-linked immunoassay, a radioimmunoassay, a fluorescent immunoassay, and a chemiluminescent immunoassay).

In the present invention, there is provided a detection for PRLR expression, including contacting a biological sample (an individual cell, a tissue, or a body fluid) using one or more PRLR antibodies (optionally coupled to a detectable portion) of the present invention, and testing if the sample shows positivity with respect to the PRLR expression, or testing if the sample has an altered (e.g., decreased or increased) expression compared to a control sample. In some embodiments, the tissue or the body fluid is a peripheral blood, a peripheral blood white blood cell, a biopsy tissue (e.g., lung or skin biopsy), and a tissue. The method can be used for a diagnostic purpose, or a non-diagnostic purpose (e.g., for PRLR pathway study, drug screening, and histochemical analysis). In some embodiments, the sample used for the non-diagnostic purpose is a cell sample, such as a cell line or an isolated cell culture.

In one embodiment, the present invention provides a method for detecting the presence of PRLR or a level thereof in a sample, including the steps of: contacting the sample with the antibody or an antigen-binding fragment thereof under conditions that allow for the formation of a complex between the antibody or the antigen-binding fragment thereof and PRLR; and detecting the formation of the complex.

In another aspect, the present invention provides a method for diagnosing a PRLR-positive disease in a subject, including the steps of: contacting an antibody or an antigen-binding fragment thereof, a polyspecific antibody, or an antibody-drug conjugate as described herein with a sample from a subject under conditions that allow for the formation of a complex between the antibody or the antigen-binding fragment thereof and PRLR; and detecting the formation of the complex, where, compared with a healthy control, the elevated level of PRLR is indicative of the presence of a disease such as a tumor or alopecia.

Optionally, the subject is a mammal, including a non-human mammal and a human. Preferably, the subject is a human.

Preferably, the tumor includes, but is not limited to: a breast cancer, a prostate cancer, an ovarian cancer, a lung cancer, a skin cancer, a pancreatic cancer, a kidney cancer, a stomach cancer, and the like.

In another aspect, the present invention provides a detecting kit, including a PRLR antigen-binding protein, an antigen-binding protein derivative and the like as described herein. In a preferable embodiment, the kit includes instructions for describing a method of use thereof.

### Embodiment 1. Preparation and screening of an antigen-immunized animal and hybridomas

### 1.1 Immunization of a mouse

A Balb/c mouse (purchased from Zhejiang Charles River Laboratory Animal Technology Co., Ltd.) were immunized subcutaneously with a commercially purchased PRLR-ECD protein (purchased from Aero Company, item No. PRR-H52Ha, an amino acid sequence as shown in SEQ ID NO: 1). On the first day, a PRLR-ECD protein emulsified with Fuchs' complete adjuvant (Sigma) was injected subcutaneously into the back of the Balb/c mouse (a human PRLR-ECD 50 µg/the mouse/0.5 mL). On the 14th day, a human PRLR-ECD protein emulsified with Fuchs' incomplete adjuvant (Sigma) was injected subcutaneously into the back of the Balb/c mouse (the human PRLR-ECD 30 µg/the mouse/0.5 mL). On the 28th day, the human PRLR-ECD protein emulsified with Fuchs' incomplete adjuvant was injected subcutaneously into the back of the Balb/c mouse (the human PRLR-ECD 30 µg/the mouse/0.5 mL). Three weeks later, 30 µg of PRLR-ECD was injected intravenously in a tail vein of the mouse for stimulation. Three to four days later, the spleen of the mouse was taken to perform fusion experiment.

### 1.2 Preparation and screening of a hybridoma cell

3-4 days after the final shock immunization of the mouse, a spleen cell of the mouse was fused with a myeloma cell SP2/0 of the mouse by a PEG method using a conventional hybridoma technical solution. The fused cells were mixed well in a complete medium (containing a RPMIl640 medium, GlutaMax, 1% Penicillin-streptomycin, 1×HAT and 20% FBS. All products were purchased from Gibco), and cultured in a total of 30 96-well culture plates at 3×10⁶ cells/200 µL/well. After 7-12 days, a supernatant was harvested. Hybridoma wells positive for human PRLR-ECD binding activity were screened by an indirect enzyme-linked immunosorbent assay (ELISA). The hybridoma wells positive for human PRLR-ECD binding were subjected to the first and second rounds of subcloning by a finite-dilution method, to obtain a target hybridoma cell strain, which was named as SPGA02-260.

The indirect enzyme-linked immunosorbent assay for screening the hybridoma wells positive for the human PRLR-ECD binding activity was performed as follows: diluting a recombinant human PRLR-ECD protein to 0.5 µg/mL with a coating solution (a phosphate coating buffer, pH 7.4), adding 100 µL/well to a enzyme-labeled plate, and coating the plate at 4°C overnight. The plate was washed once with PBST. 300 µL/well of blocking solution (1% BSA-PBST) was added. The plate was incubated at 37°C for 2 h and then washed three times with PBST for later use. A supernatant of the collected hybridoma was sequentially added to the closed enzyme-labeled plate with 100µL/well, and incubated at 37°C for 1h. The plate was washed with PBST 3 times. A HRP-labeled goat anti-mouse IgG secondary antibody (purchased from Abeam, item No.: ab6789) was added and incubate at 37°C for 30 min. After the plate was washed 3 times with PBST, residual droplets were patted to be dry as much as possible with a blotting paper. 100 µL of TMB (purchased from KPL, item No.: 52-00-03) was added to each well, placed at room temperature (20±5°C) and protected from light for 5 min. 50 µL of 2M H₂SO₄ termination solution per well was added to terminate a substrate reaction. An OD value at 450 nm of an enzyme-labeled instrument was read to analyze the binding ability of a hybridoma supernatant to be tested to a target antigen PRLR-ECD.

### Embodiment 2. The bulk preparation and determination of a murine-derived anti-human PRLR monoclonal antibody

A hybridoma cell strain obtained by amplification and screening in a serum-containing complete medium was centrifuged, and a solution is exchanged to serum-free culture-solution SFM medium to achieve a cell density of l-2×10⁷/mL. The medium was cultured for 2 weeks at 5% CO₂ at 37°C, centrifuged to obtain a culture supernatant, and purified by a protein-A affinity chromatography to obtain a murine-derived anti-human PRLR-ECD protein monoclonal antibody SPGA02-260.

### Embodiment 3. The binding activity of a murine-derived monoclonal antibody to human PRLR-ECD

An indirect enzyme-linked immunosorbent assay was used to determine the binding ability of an murine-derived antibody to human PRLR as follows: diluting a recombinant human PRLR-ECD protein to 0.5 µg/mL with a coating solution (a phosphate coating buffer, pH 7.4), adding a solution to an enzyme-labeled plate with 100 µL/well, and coating the plate at 4°C overnight. The plate was washed once with PBST. 300 µL/well of blocking solution (1% BSA-PBST) was added. The plate was incubated at 37°C for 2 h and then washed three times with PBST. The purified antibody was diluted to 1µg/mL with 1% BSA-PBST. After a solution was diluted with 1:3 gradient, the enzyme-labeled plate was added with 100µL/well, and incubated at 37°C for 1h. The plate was washed with PBST 3 times. A HRP-labeled goat anti-mouse IgG secondary antibody (purchased from Abeam, item No.: ab6789) was added and incubate at 37°C for 30 min. After the plate was washed 3 times with PBST, residual droplets were patted to be dry as much as possible with a blotting paper. 100 µL of TMB (purchased from KPL, item No.: 52-00-03) was added to each well, placed at room temperature (20±5°C) and protected from light for 5 min. 50 µL of 2M H₂SO₄ termination solution per well was added to terminate a substrate reaction. An OD value at 450 nm of an enzyme-labeled instrument was read to analyze the binding ability of the antibody to be tested to a target antigen PRLR-His.

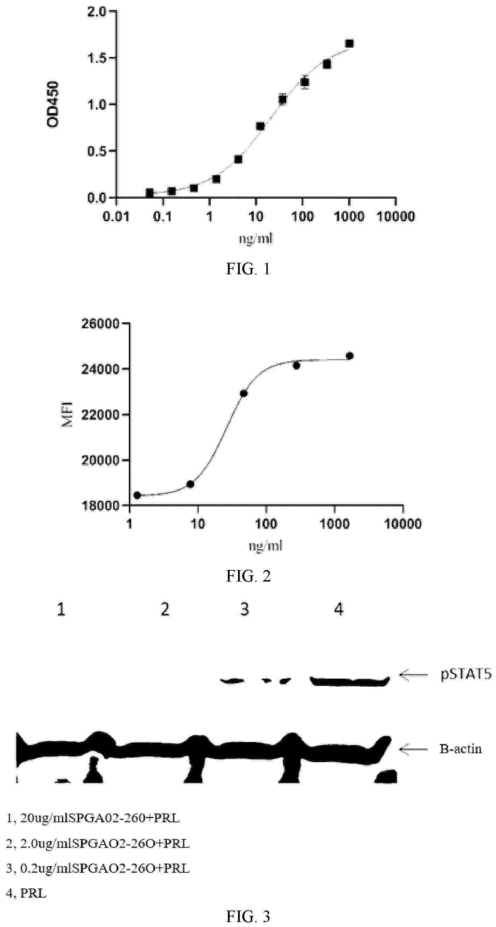

Results were shown in FIG. 1, and indicated that SPGA02-260 was bound to human PRLR-ECD with high affinity, with an EC50 of 20.9 ng/mL, i.e., 0.14 nM.

### Embodiment 4: Determination of binding affinity of a murine-derived antibody to a human breast cancer cell T47D

In this embodiment, the binding affinity of SPGA02-260 to a human breast cancer cell T47D was determined by a method of Fluorescence Activated Cell Sorting (FACS).

In this experiment, a human mammary ductal carcinoma cell T47D was used as a target cell, and SPGA02-260 was used as a primary antibody. SPGA02-260 was serially diluted from 20 µg/mL in six gradients according to a 6-fold gradient. An antibody diluted with a 100 µL gradient and 100 µL of a T47D cell were incubated for 30 min at room temperature (The cell was suspended in 100 µL of an RPMI-1640 serum-free medium (purchased from Gibco, item No.: 61870036). The maximum working concentration of a monoclonal antibody was 10 µg/mL). The cell was washed twice with PBS to remove unbound SPGA02-260. Then, the cell was incubated with 50 µL of anti-murine fluorescent secondary antibody containing 5 µg/mL Alexa Fluor 488 label (purchased from Thermo and Invitrogen, item No.: A11001) at 4°C for 40 min under light protection. The cell was washed twice with PBS to remove an unbound secondary antibody. Finally, the cell was resuspended in 100 µL of PBS. The binding affinity of SPGA02-260 to the cells was determined by a flow cytometry (purchased from Beckman, model: CytoFLEX). Data obtained were fitted and analyzed by GraphPad Prism6 software.

Results were shown in FIG. 2, the results indicated that SPGA02-260 can specifically bind a human mammary ductal carcinoma cell T47D with high expression of PRLR on a cell surface, with an EC50 of 26.0 ng/mL, i.e., 0.17 nM.

### Embodiment 5: Inhibition of PRL-induced signal transduction by a murine-derived antibody

In this embodiment, a Western blot method was used to determine the effect of an antibody SPGA02-260 on downstream transcription activator family STATS and ERK1/2 signal pathways after a human breast cancer cell T47D was stimulated with PRL.

A breast cancer cell T47D in a logarithmic growth phase was trypsin-digested, resuspended in a complete medium, spread in a 6-well plate according to 2×10⁶ Cells/ImL/well, and cultured overnight at 37°C in a CO₂ cell culture incubator. After the cell is attached to the wall of the 6-well plate on the following day, the medium was removed from the 6-well plate. 2 mL of a sterile PBS under room temperature was gently added to each well to wash away a residual medium. The PBS was removed. SPGA02-260 was diluted with a 1640 medium without a fetal bovine serum to concentrations of 20 µg/mL, 2 µg/mL and 0.2 µg/mL. 1 mL of the above antibody at different concentrations was slowly added to the cell in each well, and incubated in an incubator for 30 min. Then, 500 µL/well of 500 ng/mL of PRL (ACRO Corporation, Cat#PRN H5257) solution (prepared in a 1640 medium) was slowly added. After addition, the 6-well plate was shaken gently and placed in a CO₂ incubator to continue incubation. After 30 min of action, the 6-well plate was placed on ice. The medium was removed. Each well was gently added with 2 mL of a sterile pre-cooled PBS on ice and washed once The PBS was removed. 200 µL of RIPA lysis buffer (TEKNOVA, Cat#R3792) was added to each well, mixed well and set aside for lysis for 10 min. A cell lysis solution in the well plate was collected into a centrifuge tube, and centrifuged at 13,000 rpm for 10 min. A supernatant was extracted, subjected to BCA quantification, and then frozen into a -80°C freezer for later use. Alternatively, phosphorylated STST5 (pSTAT5) and phosphorylated ERK1/2 (pERKl/2) were detected by a conventional Western blot method.

Detection of pSTAT5: The above cell lysfis solution was subjected to SDS-PAGE electrophoresis (100 µg/lane), then transferred to a PVDF membrane by an electro-transfer method, closed in 1% BSA-TBST (shaken at room temperature for 1 h), added with 1 µg/mL (diluted in 1% BSA-TBST) of rabbit anti-human phosphorylated STATS (Tyr694) (purchased from CST. Cat#94205S), shaken and incubated at room temperature for 2h, washed with TBST for 3 times, added with a HRP-labeled goat anti-rabbit IgG secondary antibody (purchased from Abeam, Cat#ab97051, diluted 10,000-fold with 1% BSA-TBST according to instructions), shaken and incubated at room temperature for 1h, and washed with TBST for 3 times. An appropriate amount of a Pierce^{™} ECL Western blotting substrate solution (purchased from Thermo, item No. 32209) was then dropwise added onto the PVDF membrane. The PVDF membrane was protected from light at room temperature and automatically imaged on a biomolecular imager (purchased from Thermo, model: CL1500).

Detection of pERK1/2: The above cell lysis solution was subjected to SDS-PAGE electrophoresis (100 µg/lane), then transferred to the PVDF membrane by the electro-transfer method, closed in 1% BSA-TBST (shaken at room temperature for 1 h), added with 1 µg/mL (diluted in 1% BSA-TBST) of a rabbit anti-Phospho-p44/42 MAPK (Erk1/2, Thr202/Tyr204) antibody (Cell Signaling Technology company, Cat#9101), shaken and incubated at room temperature for 2h, washed with TBST for 3 times, added with the HRP-labeled goat anti-rabbit IgG secondary antibody (purchased from Abeam, Cat#ab97051, diluted 10,000-fold with 1% BSA-TBST according to instructions), shaken and incubated at room temperature for 1h, and washed with TBST for 3 times. An appropriate amount of the Pierce^{™} ECL Western blotting substrate solution (purchased from Thermo, item No. 32209) was then drop wise added onto the PVDF membrane. The PVDF membrane was protected from light at room temperature and automatically imaged on the biomolecular imager (purchased from Thermo, model: CL1500).

Detection of β-actin: The above cell lysis solution was subjected to SDS-PAGE electrophoresis (100 µg/lane), then transferred to a PVDF membrane by an electro-transfer method, closed in 1% BSA-TBST (shaken at room temperature for 1 h), added with 1 µg/mL (diluted in 1% BSA-TBST) of mouse anti-human β-actin monoclonal antibody (purchased from Thermo. Cat#MAI-140), shaken and incubated at room temperature for 2h, washed with TBST for 3 times, added with a HRP-labeled goat anti-mouse IgG secondary antibody (purchased from Beijing Biodragon Immunity Technology Co., Ltd, Cat#BF03001, diluted 10,000-fold with 1% BSA-TBST according to instructions), shaken and incubated at room temperature for 1h, and washed with TBST for 3 times. An appropriate amount of the Pierce^{™} ECL Western blotting substrate solution (purchased from Thermo, item No. 32209) was then drop wise added onto the PVDF membrane. The PVDF membrane was protected from light at room temperature and automatically imaged on the biomolecular imager (purchased from Thermo, model: CL1500).

Results were shown in FIG. 3. β-actin content was consistent across samples tested as a control. In the experiment, STATS was not phosphorylated under the conditions that a T47D cell was not stimulated by PRL, but STATS was significantly phosphorylated in the presence of PRL. SPGA02-260 antibody could completely inhibit the phosphorylation of STATS induced by PRL stimulation at concentrations of 20 µg/mL and 2 µg/mL, and still partially inhibited the phosphorylation of STATS at a concentration of 0.2 µg/mL.

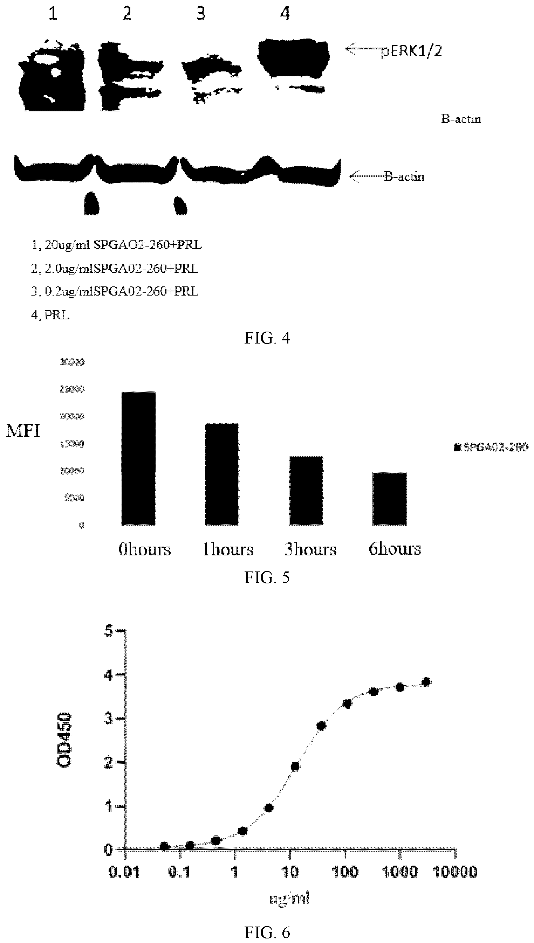

Results were shown in FIG. 4. β-actin content was consistent across samples tested as a control. In the experiment, ERK1/2H01R13/6581 was not phosphorylated under the conditions that THE T47D cell was not stimulated by PRL, but ERK1/2 was significantly phosphorylated in the presence of PRL. SPGA02-260 at concentrations of 20 µg/mL, 2 µg/mL, and 0.2 µg/mL all could completely inhibit the phosphorylation of ERK1/2 induced by PRL stimulation.

### Embodiment 6: Cytophagocytosis of a murine-derived antibody by a target cell T47D

In this embodiment, the endocytosis of SPGA02-260 on a human breast cancer cell T47D was determined by a Fluorescence Activated Cell Sorting (FACS) method.

An experimental method was as follows: A T47D cell was grown in a 24-well culture plate at 3×10⁵/well. A supernatant was discarded on the next day. The 24-well culture plate was washed once with PBS. 0.5 mL of culture solution containing 10 µg/mL SPGA02-260 was added to each well. A reaction was carried out at 37°C for 0h, 1h, 3h, and 6h, respectively. The supernatant was discarded. The cell was digested with pancreatin, transferred to a centrifuge tube, washed with PBS once, and added with 5 µg/mL of an Alexa Fluor488-labeled anti-murine fluorescent secondary antibody (purchased from Thermo and Invitrogen, item No. A11001), protected from light at 4°C, incubated for 40 min, washed again with PBS, and then resuspended in 100 µL of PBS for flow-through detection.

Results were shown in FIG. 5. The results showed that endocytosis occurred upon the binding of an antibody SPGA02-260 to PRLR antigen on the surface of the T47D cell, and was correlated with the time of action. After 3 hours, about 50% of PRLRs underwent the endocytosis.

### Embodiment 7. Preparation of a chimeric antibody

In this embodiment, a heavy chain variable region and a light chain variable region of SPGA02-260 were obtained by a relevant method of molecular biology. A chimeric antibody SPGA02-ch260 was further constructed.

RNA of a SPGA02-260 hybridoma cell was extracted by Trizol. mRNA reverse transcription was performed to obtain cDNA. Subsequently, cDNA was used as a template for PCR with heavy and light chain degenerate primers of a murine-derived antibody (Antibody Engineering, Volume 1, Edited by Roland Kontermann and Stefan DübeL. A sequence of a combination primer from page 323). A PCR product (around 700 bp) was sequenced and analyzed in a kabat database. A sequence obtained were identified as a variable region sequence of a murine-derived antibody.

The specific information of relevant sequences was shown in Table 1 above.

A gene sequence of a SPGA02-260 heavy chain variable region was 360bp in length. 120 amino acid residues were encoded. An amino acid sequence thereof was shown as SEQ ID NO: 2. A nucleic acid sequence was shown as SEQ ID NO: 3. A gene sequence of the light chain variable region was 321bp in length. 107 amino acid residues were encoded. An amino acid sequence thereof was shown as SEQ ID NO: 4. A nucleic acid sequence thereof was shown as SEQ ID NO: 5.

The resulting amino acid sequence of the heavy chain variable region was spliced with a constant region of human IgG1 (an amino acid sequence as shown in SEQ ID NO: 6). The light chain variable region was spliced with a constant region of a human kappa chain (an amino acid sequence as shown in SEQ ID NO: 7). A heavy chain (an amino acid sequence as shown in SEQ ID NO: 8) and a light chain (an amino acid sequence as shown in SEQ ID NO: 9) of SPGA02-ch260 were constructed into a pcDNA3.4 expression vector, respectively, transfected with an Expi293F cell, and purified to obtain a chimeric antibody SPGA02-ch260. A molecular weight of an expressed antibody was initially determined by SDS-PAGE electrophoresis to be correct. The purity of the antibody was greater than 95%. The antibody was quantified and preserved at 4 °C for later use.

### Embodiment 8: Determination of the affinity of a chimeric antibody for a target antigen

In this embodiment, the affinity of a chimeric antibody SPGA02-ch260 for human PRLR-ECD was determined by ELISA.

An experimental method was referred to Embodiment 3.

Results were shown in FIG. 6, indicating that SPGA02-ch260 was bound to PRLR with high affinity with an EC50 of 12.9 ng/mL, i.e., 0.09 nM.

### Embodiment 9. Determination of binding affinity of a chimeric antibody to a target cell

In this embodiment, the binding affinity of a chimeric antibody SPGA01-ch260 to a human breast cancer cell T47D was determined by a FACS method.

An experimental method was referred to Embodiment 4.

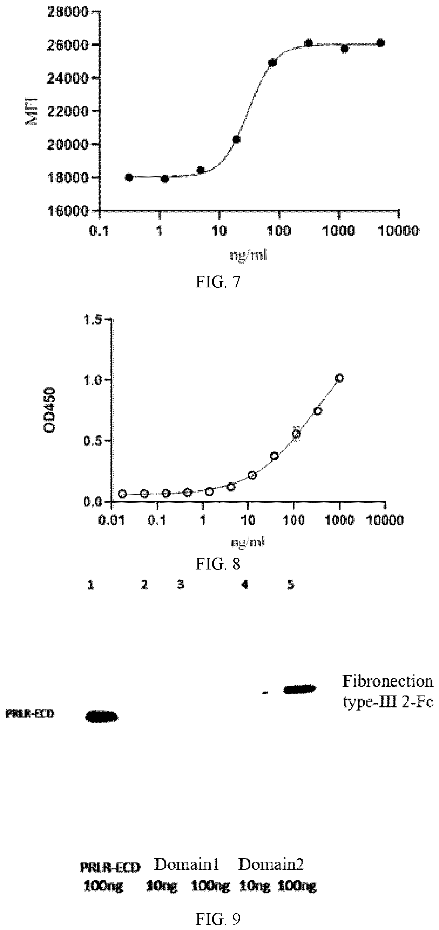

Results were shown in FIG. 7, indicating that SPGA02-ch260 was bound to a T47D cell with high expression of PRLR with high affinity, with an EC50 of 31.0 ng/mL, i.e., 0.21 nM.

### Embodiment 10. Determination of a chimeric antibody bound to mouse PRLR

In this embodiment, the difference in the binding of SPGA02-ch260 to human PRLR and mouse PRLR was determined by ELISA.

A mouse PRLR protein (purchased from Beijing Sinobiological Company, item No. 50457-M08H), i.e., mouse PRLR, was coated in a 96-well ELISA plate according to 0.5 µg/well. The cross-reactivity of SPGA02-chl8, and SPGA02-ch260 to the mouse PRLR was determined. Other specific experimental methods were referred to Embodiment 8.

Results were shown in FIG. 8, indicating that SPGA02-ch260 could be bound to a mouse PRLR protein with an EC50 of 297.3 ng/mL, which was significantly lower than the binding to human PRLR (the EC50 of 12.9 ng/mL).

### Embodiment 11. Determination of a binding region of an antibody SPGA02-260 to a target antigen

In this embodiment, an epitope of SPGA02-260 bound to PRLR was determined by a protein immunoblotting (We Stern Blot) method and ELISA.

To confirm a binding epitope of SPHA02-260 to human PRLR, an extracellular domain (PRLR-ECD) gene of human PRLR (amino acids 1 to 234 of NCBI registry number NP000940.1, an amino acid sequence as shown in SEQ ID NO: 1) was obtained by reviewing a literature and NCBI. An amino acid sequence of a functional structural domain Fibronectin type-III 1 (27-128) was as shown in SEQ ID NO: 10. An amino acid sequence of a functional structural domain Fibronectin type-III 2 (129-229) was shown in SEQ ID NO: 11. Fc fusion proteins of two functional structural domains of PRLR-ECD, i.e. Fibronectin type-III 1 and Fibronectin type-III 2, were expressed, respectively.

After being subjected to SDS-PAGE electrophoresis (400 ng/lane), reductively denatured human PRLR, and recombinantly expressed two functional structural domains, i.e., Fibronectin type-III 1 and Fibronectin type-III 2, were transferred to a PVDF membrane by an electrotransfer method, closed in 1% B SA-TB ST (shaken for 1 h at room temperature), added with a 1µg/mL murine-derived antibody SPGA02-260 (diluted with 1% BSA-TBST), shaken and incubated at room temperature for 2h, washed 3 times with TBST, then added a HRP-labeled goat anti-murine IgG secondary antibody (purchased from Abeam, item No. ab6789, diluted 10,000-fold with 1% BSA-TBST according to instructions), shaken and incubated at room temperature for 1h, and washed 3 times with TBST. An appropriate amount of Pierce^{™} ECL Western blotting substrate solution (purchased from Thermo, item No. 32209) was drop wise added onto a PVDF membrane, and the PVDF membrane was protected from light at room temperature and automatically imaged on a biomolecular imager (purchased from Thermo, Model No. CL1500).

Results were shown in FIG. 9, SPGA02-260 could specifically be bound to reductively denatured human PRLR. A second functional structural domain (i.e., Fibronectin type-III 2) of SPGA02-260-bound PRLR located at human PRLR-ECD was detected.

Human PRLR-ECD-Fibronectin type-III 2 was decomposed into 10 polypeptides overlapped each other. An amino acid sequence of each polypeptide was as follows:
1: bio-PPLELAVEVKQPEDRKPYLW(129-148);
2: bio-QPEDRKPYLWIKWSPPTLID(139-158);
3: bio-IKWSPPTLIDLKTGWFTLLY(149-168);
4: bio-LKTGWFTLLYEIRLKPEKAA(159-178);
5: bio-EIRLKPEKAAEWEIHFAGQQ(169-188);
6: bio-EWEIHFAGQQTEFKILSLHP(179-198);
7: bio-TEFKILSLHPGQKYLVQVRC(189-208);
8: bio-GQKYLVQVRCKPDHGYWSAW(199-218);
9: bio-KPDHGYWSAWSPATFIQIPS(209-228);
10: bio-SPATFIQIPSDFTMND(219-234).

The binding of SPGA02-260 to the above 10 polypeptides was determined by an ELISA method to determine a binding epitope of SPGA02-260 to PRLR.

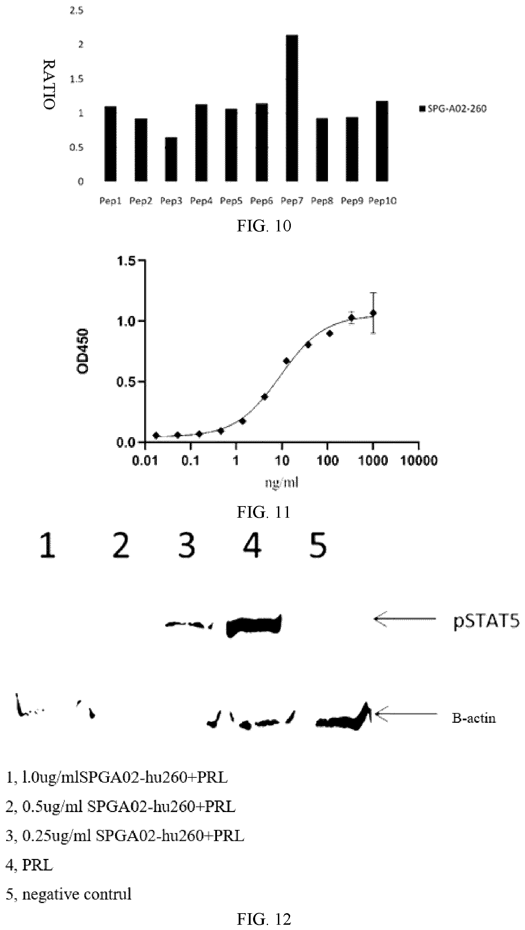

Results were shown in FIG. 10. SPGA02-260 was only bound to the above peptide 7, that is, bound to polypeptides at amino acids 189-208 at a N-terminal of a human PRLR protein, indicating that a binding epitope of SPGA02-260 was located between G189 and C208 of the human PRLR protein.

### Embodiment 12. Humanization of an antibody SPGA02-260

Amino acid sequences of a light chain variable region and a heavy chain variable region of a candidate murine-derived antibody SPGA02-260 were analyzed to determine three antigenic complementarity-determining regions (CDRs) and four framework regions (FRs) of a murine-derived antibody based on Kabat rule. Amino acid sequences of a SPGA02-260 heavy chain complementarity-determining region were HCDR1: TVSGFSLTRNGV (SEQ ID NO: 12), HCDR2: IWGDGST (SEQ ID NO: 13), and HCDR3: AKEGLYYYGRYFDV (SEQ ID NO: 14). Amino acid sequences of a light chain complementarity-determining region were LCDR1: KASQDVGSAV (SEQ ID NO: 15), LCDR2: WASTRHT (SEQ ID NO: 16) and LCDR3: QQYSNYPLT (SEQ ID NO: 17).

A humanized template with the best match to FR of each of the above murine-derived antibodies was selected from Germline database. The CDR of the murine-derived antibody was then transplanted onto the selected humanized template. The CDR of the humanized template was substituted. A variable region of a humanized antibody heavy chain (SPGA02-hu260VH, SEQ ID NO: 18) was formed and recombined with a constant region of human IgG1 (including S228P mutation, SEQ ID NO: 6) to form a humanized monoclonal antibody heavy chain (SPGA02-hu260H, SEQ ID NO: 20). A light chain variable region of the humanized antibody (SPGA02-hu260VL, SEQ ID NO: 19) was recombined with a constant region of a human kappa chain to form a humanized light chain (SPGA02-hu260L, SEQ ID NO: 21). A heavy chain and a light chain of a humanized antibody were constructed into pcDNA3.4 expression vector, respectively. An Expi-293F cell was transfected. The humanized antibody SPGA02-hu260 was obtained by the purification of Protein A. The correct molecular weight size and purity >95% of each SPGA02-hu260 antibody were determined by SDS-PAGE electrophoresis and SEC-HPLC.

### Embodiment 13. Determination of the affinity of humanized antibody SPGA02-hu260 for a target antigen

In this embodiment, the affinity of a humanized antibody SPGA02-hu260 for human PRLR-ECD was determined by an ELISA method.

An experimental method was referred to Embodiment 3.

Results were shown in FIG. 11, indicating that SPGA02-hu260 was bound to PRLR with high affinity with an EC50 of 8.98 ng/mL, i.e., 0.06 nM.

### Embodiment 14: Inhibition of signal transduction induced by binding of PRLR to PRL by a humanized antibody SPGA02-hu260

An experimental method was referred to Embodiment 5. The action concentration of an antibody SPGA02-hu260 was adjusted to 1.0/0.5/0.25/0.1 µg/mL.

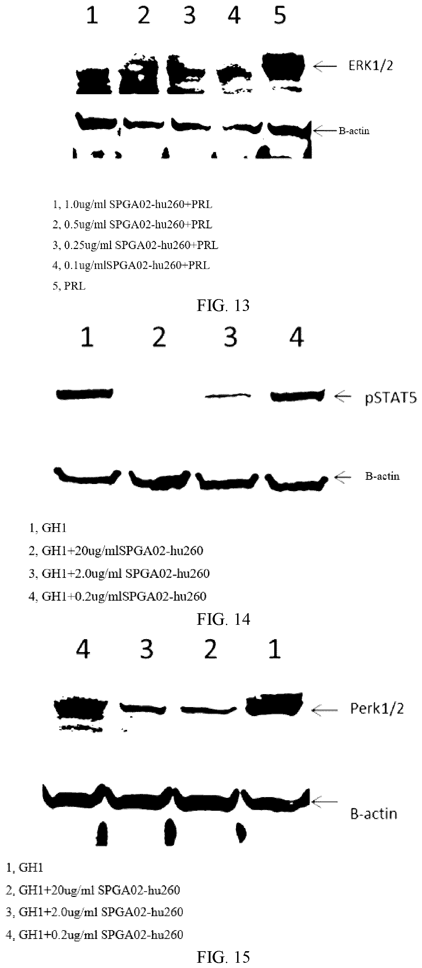

Results were shown in FIGS. 12 and 13, the content of β-actin used as a control was consistent across samples tested.

When T47D was acted by PRL, STATS was significantly phosphorylated. An antibody SPGA02-hu260 completely inhibited the phosphorylation of STATS induced by a T47D cell at PRL stimulation at concentrations of 1 µg/mL and 0.5 µg/mL, but partially inhibited the phosphorylation of STATS induced by PRL at a concentration of 0.25 µg/mL.

When T47D was acted by PRL, ERK1/2 was significantly phosphorylated. An SPGA02-hu260 antibody was still able to completely inhibit PRL-stimulated induced phosphorylation of ERK1/2 at a concentration as low as 0.1 µg/mL.

### Embodiment 15: Inhibition of signal transduction induced by binding of PRLR to GH by a humanized antibody SPGA02-hu260

An experimental method was essentially the same as that of Embodiment 5, for human PRL was substituted by a 1 µg/mL human growth hormone (GH1, SinoBiological, Cat# 16122-HNCE) as a stimulant to induce signal transduction of PRLR.

Results were shown in FIGs 14 and 15, the content of β-actin was consistent as a control in each sample tested.

When T47D was acted by GH1, STATS was significantly phosphorylated. An antibody significantly inhibited the phosphorylation of STATS in a T47D cell stimulated by GH1 at concentrations of 20 µg/mL and 2 µg/mL, whereas this inhibitory effect largely disappeared at a concentration of 0.2 µg/mL.

When T47D was acted by GH1, ERK1/2 was significantly phosphorylated. An SPGA02-hu260 antibody was still able to significantly inhibit GH1 stimulation-induced phosphorylation of ERK1/2 at concentrations as low as 0.2 µg/mL.

### Embodiment 16: Inhibition of the growth of a BAF3-huPRLR cell by an antibody SPGA02-hu260

Mouse progenitor B cell BAF3 in a logarithmic phase of growth cultured in a 1640 medium (complete medium) containing 10 ng/mL of mouse IL3 (Beyotime, P5912) and 10% FBS (Gibico) were infected with a human PRLR overexpressing lentivirus (Genomeditech (Shanghai) Co., Ltd., item No. GM-10162OL01). A cell line stably over expressing human PRLR, i.e. BAF3-huPRLR, was obtained by screening and subcloning under the condition of 5µg/mL Puromycin Dihydrochloride (Genomeditech (Shanghai) Co., Ltd., item No. GM-040401-1).

A BAF3-huPRLR cell was cultured in a 1640 cell culture solution containing 40 ng/mL of human PRL (ACROBiosystems Group, PRR-H52Ha). 2×105/100 µL were inoculated into a 96-well cell culture plate. An antibody SPGA02-hu260 was diluted to 30 µg/mL with a PRL-free 1640 culture solution, and then 10-fold diluted for a total of 8 Gradients. The above 96-well cell culture plate containing a BAF3-huPRLR cell was added with 100µL/well, respectively. The cell was cultured in a CO₂ incubator for 72 h. Then, a CCK8 reagent was added according to the instructions. OD450 was measured after incubation at 37°C for 2 h.:

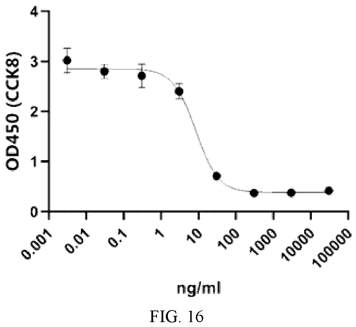

As shown in FIG. 16, SPGA02-hu260 was able to effectively inhibit the growth of a BAF3-huPRLR cell induced by the stimulation of human PRL, with an IC50 of 8.28 ng/mL, i.e., 0.06 nM.

The forgoing embodiments only express a plurality of embodiments of the present invention, and the description was relatively specific and detailed, but it should not be understood as a limitation to the patent scope of the present invention. It should be noted that for those of ordinary skill in the art, several modifications and improvements can be made without departing from the concept of the present invention and these modifications and improvements should all fall within the protection scope of the present invention. Therefore, the protection scope of the present invention shall be subject to the appended claims.

## Claims

1. A PRLR antigen-binding protein, **characterized by** comprising the following complementarity-determining regions:
(1) a heavy-chain complementarity-determining region 1HCDR1 comprising an amino acid sequence shown in SEQ ID NO: 12 or a variant sequence thereof;
(2) a heavy-chain complementarity-determining region 2HCDR2 comprising an amino acid sequence shown in SEQ ID NO: 13 or a variant sequence thereof;
(3) a heavy-chain complementarity-determining region 3HCDR3 comprising an amino acid sequence shown in SEQ ID NO: 14 or a variant sequence thereof;
(4) a light-chain complementarity-determining region 1LCDR1 comprising an amino acid sequence shown in SEQ ID NO: 15 or a variant sequence thereof;
(5) a light-chain complementarity-determining region 2LCDR2 comprising an amino acid sequence shown in SEQ ID NO: 16 or a variant sequence thereof;
(6) a light-chain complementarity-determining region 3LCDR3 comprising an amino acid sequence shown in SEQ ID NO: 17 or a variant sequence thereof.

2. The antigen-binding protein according to claim 1, **characterized in that** the antigen-binding protein comprises:
(1). a heavy-chain variable region VH comprising an amino acid sequence shown in SEQ ID NO:2; and/or a light-chain variable region VL comprising an amino acid sequence shown in SEQ ID NO:4;
or (2). a VH having at least 70%, at least 80%, at least 85%, at least 90%, at least 91%, at least 92%, at least 93%, at least 94%, at least 95%, at least 96%, at least 97%, at least 98%, or at least 99% sequence identity as compared to the VH in (1); and/or, a VL having at least 70%, at least 80%, at least 85%, at least 90%, at least 91%, at least 92%, at least 93%, at least 94%, at least 95%, at least 96%, at least 97%, at least 98%, or at least 99% sequence identity compared to the VL in (1);
or (3). a VH having substitution, deletion or addition of one or several amino acids or any combination thereof as compared to the VH in (1); and/or, a VL having substitution, deletion or addition of one or several amino acids or any combination thereof as compared to the VL in (1), the substitution being the conservative substitution.

3. The antigen-binding protein according to claim 2, **characterized in that** the antigen-binding protein further comprises a human IgG1 constant region and a human kappa-chain constant region, the human IgG1 constant region has an amino acid sequence as shown in SEQ ID NO: 6, the human kappa-chain constant region has an amino acid sequence as shown in SEQ ID NO: 7, a VH of the PRLR antigen-binding protein is linked to the human IgG1 constant region to form a heavy chain, and a VL of the PRLR antigen-binding protein is linked to the human kappa chain constant region to form a light chain.

4. The antigen-binding protein according to claim 3, **characterized in that** the antigen-binding protein comprises:
(1) a heavy chain HC comprising an amino acid sequence shown in SEQ ID NO:8; and/or a light chain LC comprising an amino acid sequence shown in SEQ ID NO:9;
or (2) a heavy chain and a light chain, wherein the heavy chain has at least 80%, at least 85%, at least 90%, at least 91%, at least 92%, at least 93%, at least 94%, at least 95%, at least 96%, at least 97%, at least 98%, or at least 99% sequence identity as compared to the heavy chain and the light chain in (1); and/or, the light chain has at least 70%, at least 80%, at least 85%, at least 90%, at least 91%, at least 92%, at least 93%, at least 94%, at least 95% , at least 96%, at least 97%, at least 98% or at least 99% sequence identity.

5. A humanized PRLR antigen-binding protein, **characterized by** comprising:
(1). a heavy chain variable region VH comprising an amino acid sequence shown in SEQ ID NO:18; and/or a light chain variable region VL comprising an amino acid sequence shown in SEQ ID NO:19:
or (2). a VH having at least 70%, at least 80%, at least 85%, at least 90%, at least 91%, at least 92%, at least 93%, at least 94%, at least 95%, at least 96%, at least 97%, at least 98%, or at least 99% sequence identity as compared to the VH in (1); and/or, a VL having at least 70%, at least 80%, at least 85%, at least 90%, at least 91%, at least 92%, at least 93%, at least 94%, at least 95%, at least 96%, at least 97%, at least 98%, or at least 99% sequence identity compared to the VL in (1);
or (3). a VH having substitution, deletion or addition of one or several amino acids or any combination thereof as compared to the VH in (1); and/or, a VL having substitution, deletion or addition of one or several amino acids or any combination thereof as compared to the VL in (1), the substitution being the conservative substitution.

6. The humanized PRLR antigen binding protein according to claim 5, **characterized in that** the humanized PRLR antigen binding protein comprises:
(1) a heavy chain HC comprising an amino acid sequence shown in SEQ ID NO:20; and/or a light chain LC comprising an amino acid sequence shown in SEQ ID NO:21;
or (2) a heavy chain and a light chain, where the heavy chain has at least 80%, at least 85%, at least 90%, at least 91%, at least 92%, at least 93%, at least 94%, at least 95%, at least 96%, at least 97%, at least 98%, or at least 99% sequence identity as compared to the heavy chain and the light chain in (1); and/or, the light chain has at least 70%, at least 80%, at least 85%, at least 90%, at least 91%, at least 92%, at least 93%, at least 94%, at least 95%, at least 96%, at least 97%, at least 98% or at least 99% sequence identity.

7. A nucleic acid molecule for encoding the PRLR antigen-binding protein according to any one of claims 1-4 or the humanized PRLR antigen-binding protein according to any one of claims 5-6.

8. A vector comprising the nucleic acid molecule of claim 7.

9. A host cell comprising the nucleic acid molecule according to claim 7 or the vector according to claim 8.

10. A pharmaceutical composition **characterized by** comprising the PRLR antigen-binding protein according to any one of claims 1-4, the humanized PRLR antigen-binding protein according to any one of claims 5-6, the nucleic acid molecule according to claim 7, the vector according to claim 8, and/or the host cell according to claim 9.

11. Use of the PRLR antigen-binding protein according to any one of claims 1-4, the humanized PRLR antigen-binding protein according to any one of claims 5-6, the nucleic acid molecule according to claim 7, the vector according to claim 8, the host cell according to claim 9, and/or the pharmaceutical composition according to claim 10 in preparation of a drug for preventing and/or treating a PRLR-positive disease, a kit and/or an administrating device.

12. The use according to claim 11, **characterized in that** the PRLR-positive disease is a tumor or alopecia.
